(19) **Europäisches Patentamt / European Patent Office / Office européen des brevets**

(11) **EP 1 965 224 B1**

(12) **EUROPEAN PATENT SPECIFICATION**

(45) Date of publication and mention
of the grant of the patent:
**27.01.2010 Bulletin 2010/04**

(51) Int Cl.:
*G01S 15/89* (2006.01)   *G01S 7/52* (2006.01)
*A61B 8/00* (2006.01)

(21) Application number: **07022428.2**

(22) Date of filing: **19.11.2007**

(54) **Ultrasonic imaging apparatus**

Ultraschallabbildungsvorrichtung

Appareil de formation d'images à ultrasons

(84) Designated Contracting States:
**DE FR GB IT**

(30) Priority: **27.02.2007   JP 2007046390**

(43) Date of publication of application:
**03.09.2008   Bulletin 2008/36**

(60) Divisional application:
**09005375.2 / 2 081 051**

(73) Proprietor: **Hitachi, Ltd.**
**Chiyoda-ku**
**Tokyo 100-8280 (JP)**

(72) Inventors:
• **Azuma, Takashi**
**Chiyoda-ku**
**Tokyo 100-8220 (JP)**
• **Kawabata, Kenichi**
**Chiyoda-ku**
**Tokyo 100-8220 (JP)**

(74) Representative: **Strehl Schübel-Hopf & Partner**
**Maximilianstrasse 54**
**80538 München (DE)**

(56) References cited:
**EP-A- 1 406 096**   **EP-A- 1 723 911**
**WO-A-99/35967**   **US-A- 5 833 614**
**US-A1- 2005 273 010**

• **STRIDE E ET AL: "MICROBUBBLE ULTRASOUND CONTRAST AGENTS: A REVIEW" PROCEEDINGS OF THE INSTITUTION OF MECHANICAL ENGINEERS. JOURNAL OF ENGINEERING IN MEDICINE. PART H, MECHANICAL ENGINEERING PUBLICATIONS LTD, LONDON, GB, vol. 217, no. H6, 1 November 2003 (2003-11-01), pages 429-447, XP008078024 ISSN: 0954-4119**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

FIELD OF THE INVENTION

The present invention relates to an apparatus for displaying an ultrasonic tomogram.

DESCRIPTION OF THE RELATED ART

It has been a long time since an image diagnostic modality such as an X-ray computed tomography (X-ray CT) scanner, a magnetic resonance imaging (MRI) machine and an ultrasonic diagnostic apparatus became an essential equipment in the medical site. These image a difference in CT value, spin relaxation time and acoustic impedance in living organisms, respectively, and are called "morphology imaging", because the difference in these physical characteristics reflects mainly an organic structure (shape). On the contrary, an imaging for imaging a site of a tissue that is structurally the same, but functionally in a different state is called "function imaging". In the field of function imaging, an imaging for visualizing a presence state of a living component especially such as protein is often called "molecular imaging". The molecular imaging is a research field that now most attracts attention because it is expected to be applicable to clarifying a life phenomenon such as development and differentiation and diagnosing and treating a disease. In the molecular imaging, a "molecular probe" that is material having a structure for selecting a living component is often used. In this case, a structure that can make the molecular probe to be detected by some physical means is added, visualizing distribution of the molecular probe in vivo.

If, as the physical means for detecting the molecular probe, a diagnostic imaging apparatus for medical care can be used, conventional morphology imaging and molecular imaging can be fused together, and it is expected that an advanced treatment can be provided. Important technologies in performing molecular imaging using ultrasonic waves includes a technology for visualizing distribution of the molecular probe in vivo on an ultrasonic image. Generally, it is difficult for design to provide the molecular probe itself with sufficient sensitivity to appear on an ultrasonic diagnostic apparatus. Accordingly, a contrast agent that chemically or physically combines with the molecular probe to visualize a presence state of the molecular probe on an image diagnostic apparatus is used in combination with the molecular probe, allowing for the optimal design for each of the molecular probe and the contrast agent, and a large degree of freedom for development. Currently, a generally used ultrasonic contrast agent is a microbubble having a diameter of several micrometers. An ultrasonic diagnostic apparatus has features that a site different in acoustic impedance of a substance, that is, a physical value of density multiplied by the speed of sound is visualized, and it is easy to visualize, in vivo, a microbubble having acoustic impedance of about $0.004 \times 10^6$ kg/m$^2$·s (air) extremely smaller than bodily acoustic impedance of about $1.5 \times 10^6$ kg/m$^2$·s. Also, the microbubble of several micrometers resonates with ultrasonic waves in the band of several MHz used for diagnosing, and creates a harmonic component of the irradiated ultrasonic waves, so that only the signal from the microbubble can be selectively visualized, allowing for more sensitive imaging.

It is quite expected that, to realize the molecular imaging using ultrasonic waves, an agent having a structure in which a molecular probe is combined with a microbubble already used for diagnosing blood flow, as described above, is used as a contrast agent used with the molecular probe. For example, as disclosed in Patent Document 1 see beneath, an agent has been developed as a blood clot selecting contrast agent. Further, for example, as disclosed in Non-patent Document 1 see beneath, a new blood vessel selecting contrast agent has been also developed. However, a contrast agent using the micron-sized bubble has disadvantages that it is difficult to image other than the blood vessel and an applicable range is limited. Further, the microbubble is destroyed even at the diagnosing level of ultrasound intensity, and because the microbubble is destroyed once imaged, it is difficult to continuously image. Also, because the microbubble is a bubble (gas), the microbubble is discharged due to gas exchange in the lungs, presenting a problem that the microbubble can stay in the blood only for ten-odd minutes.

On the contrary, for example as shown in Non-patent Document 2 see beneath, a research concerning a contrast agent has been also conducted that acoustic impedance capsules liquid different from living organisms to form a submicron sized bubble, allowing for transition from the blood vessel to the tissue. According to this approach, it is thought that application to a site other than the blood vessel may be allowed. Further, it is expected that a staying time in the blood is longer than that of a gas. However, because finely formed liquid is used, resonance which occurs in the case of the microbubble contrast agent described above does not occur, causing a disadvantage in terms of sensitivity.

As a contrast agent having advantages of these approaches together, as shown in Patent Document 2 see beneath, a type of contrast agent has been studied, in which a compound that is a fine particle formed of liquid by a surface active agent is dosed to and vaporized inside the body with irradiation of ultrasonic waves. This type of contrast agent has less restrictions concerning the staying time in the body and an applicable site, and also, because of visualization in a state of gas, it is thought to be possible to image at high sensitivity using resonance.

In the case of the type of contrast agent in which the compound that is a fine particle formed of liquid by a surface active agent is dosed to and vaporized inside the body with irradiation of ultrasonic waves, as a method for specifically imaging a signal from a microbubble of several micrometers after being vaporized, a method using a received echo having a different frequency from a transmission frequency is disclosed, for example, in Patent Documents 3, 4.

[Patent Document 1] U.S. Patent No. 6521211
[Patent Document 2] U.S. Patent No. 5716597
[Patent Document 3] JP-A-11-137547
[Patent Document 4] JP-A-2003-135467

[Non-patent Document 1] Ellegala et al. Circulation 108: 336-341 (2003)
[Non-patent Document 2] Lanza et al. Circulation 94: 3334-3340 (1996)

An overview over ultrasonic imaging with microbubble contrast agents is given by Stride et al. in: "Microbubble ultrasound contrast agents: a review", Proceedings of the Institution of Mechanical Engineers, Journal of Engineering in Medicine, part H, Mechanical Engineering Publications Ltd., London, GB, vol. 217, No. H6, November 2003, pages 429 to 447. The authors mention the use of harmonic components in the echo signal to distinguish echoes due to the contrast agent from those due to tissue. US 5,833,614 discloses ultrasonic imaging by use of harmonic echo signals. The document discloses transmit waveforms intended to enhance generation of harmonic response in a contrast agent or tissue. US 2005/0,273,010 Al and EP 1 723 911 Al disclose filters to restrict the frequency band of the received signals.

SUMMARY OF THE INVENTION

Considering imaging in which morphology imaging and molecular imaging are fused together, it is important to realize a system using ultrasonic waves. One reason is a high real time property. The morphology/component fused imaging is thought to be able to quite early detect a disease, but in such quite early stage, a disease area is small, so that there may be often a situation that it can be determined only that there may be the disease area, based on only one image. Under such situation, it is thought to be important to observe an area of interest from diverse angles. For such purpose, ultrasonic waves having the superior real time property may form a superior tool. The second reason may be that ultrasonic waves can work for both diagnosing and curing by changing irradiation conditions, and further have a quite weak invasiveness into living organisms when both diagnosing and curing. As described above, according to the morphology/component fused imaging, it is thought to be possible to quite early diagnose a disease and it is expected that a treatment object has an extremely small, restricted area. Under such situation, a treatment method having a high invasiveness entails too much risk, and treatment having a weak invasiveness is desirable. Using ultrasonic waves, it is thought to be possible to diagnose and also perform treatment having a weak invasiveness on the site.
However, in a system using ultrasonic waves, when an ultrasound probe has a finite band, to separate a band of a transmission waveform and a band of a received waveform from a contrast agent, at least one of a transmission band and a reception band becomes narrow, lowering space resolution. That is, it has been difficult to satisfy both a discrimination factor of the signal from the contrast agent to another signal (specificity of the contrast agent signal) and the space resolution.
It is, therefore, an object of the invention to provide an ultrasonic imaging apparatus which can discriminate a signal specific to a contrast agent from other signals and can produce an image of high resolution.
This object is solved by an apparatus in accordance with claim 1. The dependent claims relate to preferred embodiments of the invention.
The present invention is rather than that a liquid formed in a fine particle is once vaporized to turn into a contrast agent, and subsequently, an ultrasonic pulse is sent to visualize it, that a signal peculiar to a contrast agent emitted when a liquid formed in a fine particle vaporizes due to ultrasonic waves and the imaging space distribution thereof is detected. When the liquid formed in a fine particle is vaporized, it is necessary for energy to accumulate, requiring for a waveform of the ultrasonic waves to be long to some extent and resulting in a narrow signal in the frequency band. An echo signal to this long waveform has a narrow band for both a fundamental wave and a harmonic wave. On the one hand, the ultrasonic waves emitted when vaporized are short in the time axis because of instantaneous vaporization. That is, in the frequency domain, a waveform having a wide band is formed. In the present invention, because a narrow band can be used for transmission and a wide band can be used for a received signal, it is possible to discriminate a transmission signal and a received signal in a state of high space resolution in the received signal.
An ultrasonic imaging apparatus, as one example, but not part of the present invention, includes: transmission means for transmitting a first ultrasonic signal to an object area of a subject; receiving means for receiving, from the subject, a second ultrasonic signal generated due to irradiation of the first ultrasonic signal; and a computing portion for filtering out at least one of a fundamental pulse and a harmonic pulse in the first ultrasonic signal from the second ultrasonic signal to compute a detection signal.

An ultrasonic imaging apparatus according to the present invention includes: transmission means for transmitting a first ultrasonic signal and a second ultrasonic signal having a cycle number larger than that of the first ultrasonic signal to a phase transition contrast agent dosing area of a subject; receiving means for receiving, from the subject, a third ultrasonic signal and a fourth ultrasonic signal respectively generated due to the first ultrasonic signal and the second ultrasonic signal; and a computing portion for processing the third ultrasonic signal so that the cycle number thereof coincides with that of the second ultrasonic signal, to form a fifth ultrasonic signal, and computing a difference between the fourth ultrasonic signal and the fifth ultrasonic signal as a detection signal.

It is possible to satisfy both a discrimination factor of a signal peculiar to a contrast agent to another signal (specificity of the contrast agent signal) and space resolution.

Other objects, features and advantages of the invention will become apparent from the following description of the embodiments of the invention taken in conjunction with the accompanying drawings.

BRIEF DESCRIPTION OF THE DRAWINGS

Fig. 1 is a block diagram of an example of an ultrasonic imaging apparatus;
Figs. 2A, 2B describe a method for imaging a contrast agent that generates microbubbles based on a conventional concept;
Figs. 3A, 3B describe a method for concurrently generating microbubbles and taking a contrast image not forming part of the present invention;
Fig. 4 describes a waveform in the time axis and a frequency band of a transmission pulse and an emission pulse;
Fig. 5 is a flow chart of sequences for detecting an emission pulse signal from a contrast agent;
Figs. 6A, 6B describe a trade off between a discrimination factor of an echo from the tissues and a imaging signal, and a space resolution in a conventional method;
Figs. 7A, 7B show experimental data of a threshold value for bubble generation to a pulse wavelength;
Fig. 8 is a graph illustrating the relation between a pulse cycle number and a relative bandwidth, when a Hanning function is used as an envelope;
Fig. 9 is a flow chart illustrating an embodiment of the invention;
Fig. 10 describes a waveform in this embodiment;
Fig. 11 describes a band of a probe; and
Fig. 12 is a block diagram of an example of an apparatus according to the present invention.

DETAILED DESCRIPTION OF THE INVENTION

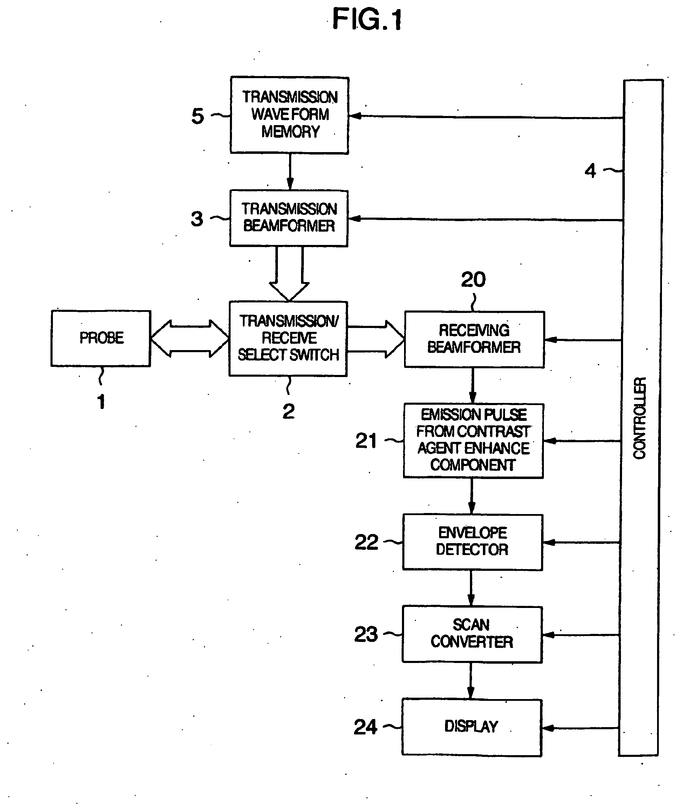

First, referring to Fig. 1, a flow of processing a signal for imaging in an ultrasonic diagnostic apparatus will be described. A transmission electric pulse is sent from a transmission beamformer 3 to an ultrasonic probe 1 provided on a surface of a subject (not shown) through a transmission/receive select switch 2 under control of a controller 4. The transmission/receive select switch 2 switches connection between transmission means and receiving means of ultrasonic waves (not shown), and the ultrasonic probe 1. At this time, the transmission beamformer suitably controls a delay time between each channel of the probe 1 so that an ultrasonic beam proceeds along a desired scanning line. The ultrasonic probe 1, receiving an electric signal from the transmission beamformer 3, converts the electrical signal into an ultrasonic signal to send an ultrasonic pulse into the subject. The ultrasonic pulse is scattered in the subject, and a part of it is again received as an echo signal and converted from an ultrasonic signal into an electrical signal by the ultrasonic probe 1. This received signal, through the transmission/receive select switch 2, passes to a receiving beamformer 20, and an echo signal from a desired depth in the desired scanning line is selectively enhanced by the receiving beamformer 20 to provide data on some scanning line. An RF signal on this scanning line has a signal peculiar to an contrast agent enhanced by an emission pulse from contrast agent enhance component 21, is converted into an envelope signal and log-compressed in an envelope detector 22, and sent to a scan converter 23. The scan converter 23 scans and converts it into a video signal. Data after this scan-conversion is sent to a display 24 to be displayed as an ultrasonic tomogram. Except transmission waveform and the emission pulse from the contrast agent enhance component, here, detailed description will be omitted.

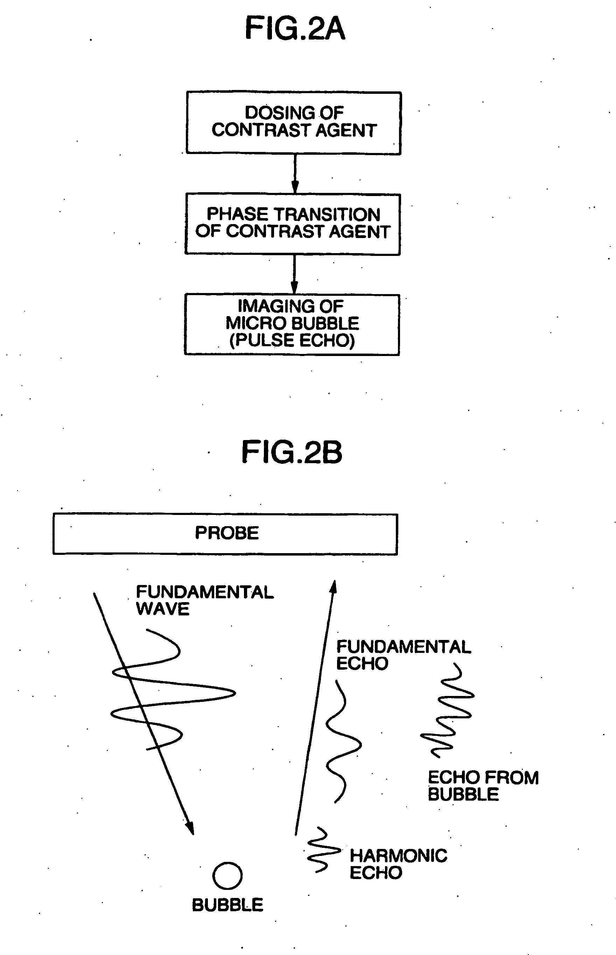

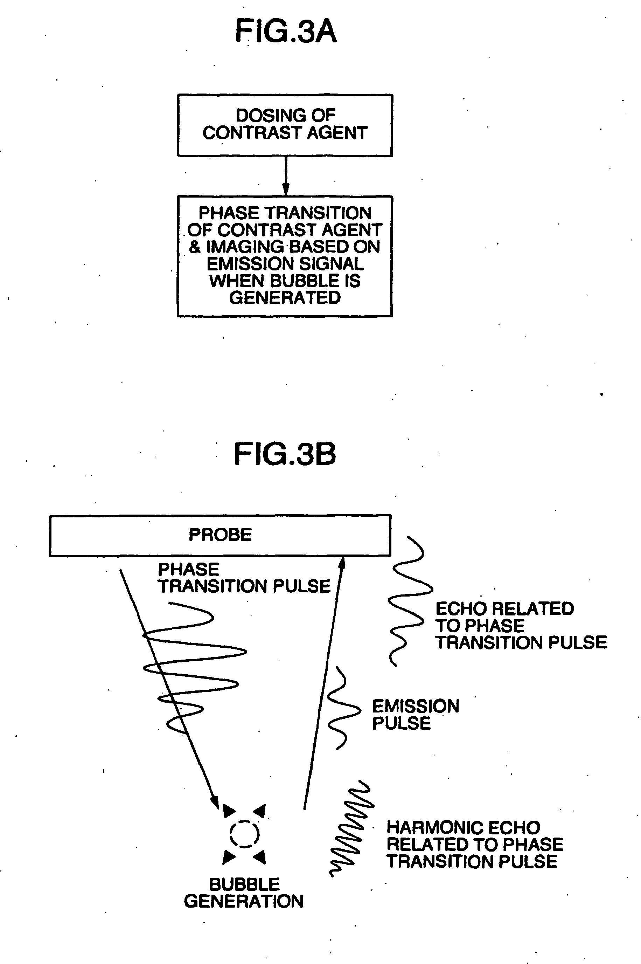

Referring to Figs. 2A, 2B, and 3A, 3B, a flow of ultrasonic irradiation will be described, compared with a conventional method. According to the conventional method shown in Fig. 2A, first, a contrast agent is dosed to a subject. Next, when the contrast agent has circulated to some extent, ultrasonic waves are irradiated to generate microbubbles from the contrast agent in an area of interest. After bubble generation, according to general sequences for imaging a microbubble, an image is taken to form a contrast image. Fig. 2B shows a waveform produced according to this series of sequences. The ultrasonic probe radiates a fundamental wave, and subsequently, three types of signal in an area having microbubbles: an echo from a microbubble; a fundamental echo reflected by the tissues around; and a harmonic echo also reflected by the tissues around, are returned to the ultrasonic probe. In this case, it is necessary to selectively detect only the echo from a microbubble among the three types of received signal.

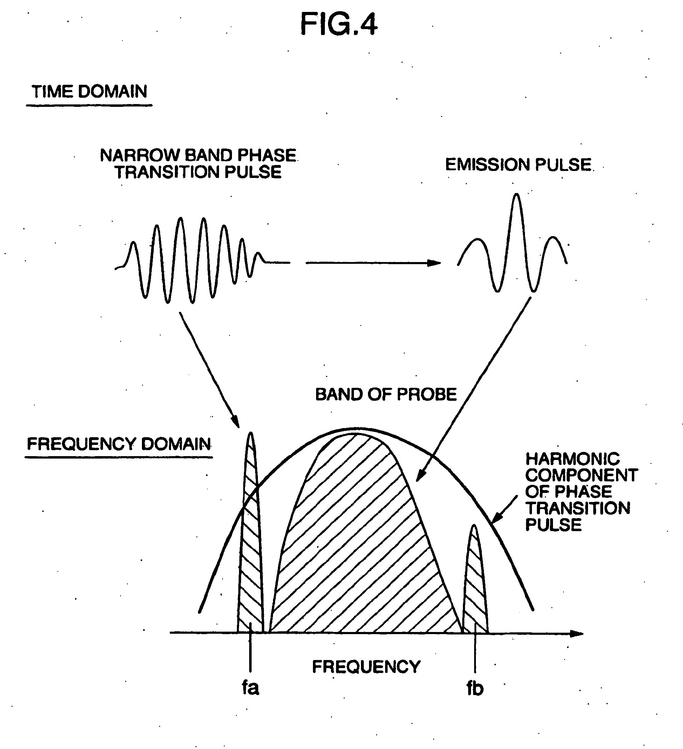

On the contrary, according to the example shown in Fig. 3A, after dosing of a contrast agent, bubble generation and imaging are concurrently performed. In this case, as shown in Fig. 3B, a phase transition (bubble generation) pulse is radiated, and subsequently, three types of signal in an area having the contrast agent: ultrasonic waves emitted at the time of bubble generation (hereinafter, called "emission pulse"); a fundamental echo related to the phase transition pulse reflected by the tissues around; and a harmonic echo also reflected by the tissues around, are returned to the ultrasonic probe. The emission pulse is an ultrasonic wave that is radiated around by the contrast agent itself at the time of bubble generation from the contrast agent. Fig. 3B schematically shows by the arrow that the ultrasonic wave is irradiated at the time of the bubble generation. These three signal components are discriminated by a frequency band. Referring to Fig. 4, the relation between the three pulses described above in the frequency axis will be described. The phase transition pulse is set to be longer in the time axis. Accordingly, it becomes narrow in the frequency domain. In the case where a transmission pulse has a narrow band, a harmonic pulse has also a narrow frequency band. On the contrary, the emission pulse is radiated in a broad band independent of this. Each of a fundamental pulse of the phase transition pulse and a harmonic pulse such as a second harmonic pulse is set to be positioned at both ends (both ends or in the vicinity of them) of a band of the ultrasonic probe, and thereby the emission pulse can be dealt with by using a broad, central portion of the band.

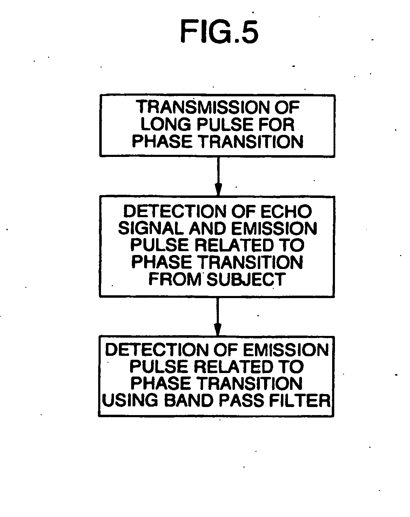

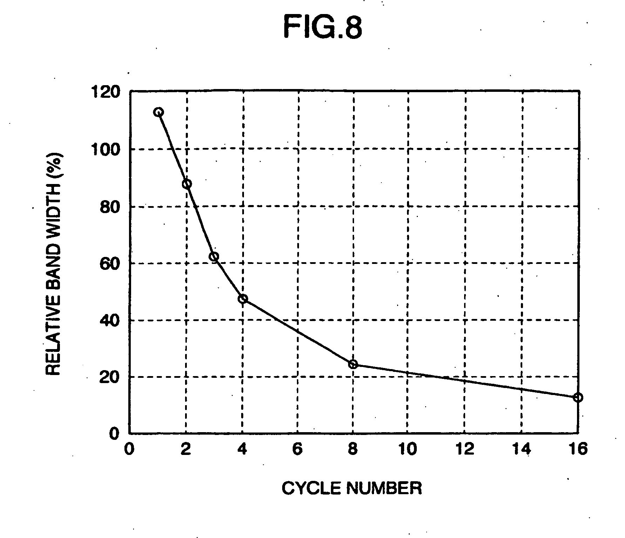

Fig. 5 shows a flow chart that organizes the series of sequences. Processes shown in Fig. 5 are carried out in the emission pulse from contrast agent enhance component in Fig. 1 (computing portion for computing). There are a number of methods for realizing a band pass filter. Here, a method for realizing by combining frequency shift and a Hanning function which is a low pass filter will be described. Using the Hanning function as the low pass filter, a width of the Hanning function is determined relative to a desired bandwidth in the low pass filter as shown in Fig. 8. The frequency shift is accomplished by multiplying a received signal by a sin wave of a shift frequency f. Let the received signal be $\sin(f1 \times t)$, multiplying this by $\sin(f \times t)$, the trigonometric theory concerning the sum of products gives $\sin(f1 \times t) \times \sin(f \times t) = -0.5 \times \{\cos((f1 + f) \times t) - \cos((f1 - f) \times t)\}$. This signal is passed through the low pass filter described above, filtering out a high frequency component $\cos((f1 + f) \times t)$, and providing a signal having a frequency of f1 - f. That is, a signal having a frequency lowered by f is formed.

In such a manner, a center frequency f of the band pass filter and a relative bandwidth are determined, and then a signal only in a window portion in the frequency band set to the received signal can be detected, so that the phase transition pulse used for bubble generation and this harmonic component, and the emission pulse transmitted at the time of contrast agent vaporizing can be discriminated.

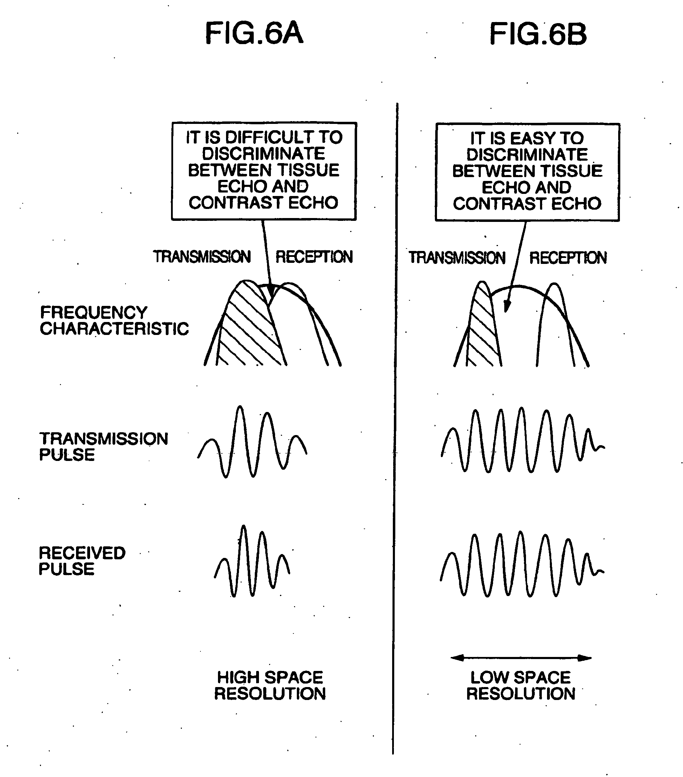

Superiority of the configuration will be hereinafter described, compared with a conventional method shown in Figs. 6A, 6B. In the case shown in Fig. 6A, for both the transmission and reception, three waves (cycles) of sin are used in which a Hanning function is an envelope, and as can be seen apparently from Fig. 8, the relative bandwidth is 60%. In this case, the relative bandwidth of the current ultrasonic probe is about 70%, and bands of transmission and reception almost overlap with each other. For example, when the band of the probe is 2 MHz to 4.2 MHz, to set the relative bandwidth for a transmission pulse to 60%, the band requires 2 MHz to 3.7 MHz, so that the band of the probe is almost used. Accordingly, to use a received pulse in the band of the probe, the band requires 2.25 MHz to 4.2 MHz, resulting in the relative bandwidth of 60%, and in the range from 2.25 MHz to 3.7 MHz, the bands for transmission and reception overlap with each other.

On the one hand, if the band for reception is set not to overlap, the band cannot be included in the band of the probe, as the result, sensitivity in reception becomes poor. In the case shown in Fig. 6B, the relative bandwidth of transmission and reception is 25%, and the pulse waveform has eight waves of sin from Fig. 8. The sum of the relative bandwidths is 50%, and included in 70% of the band of the probe. However, when the pulse waveform has eight waves, compared with a conventional diagnostic apparatus, space resolution becomes worse about three times. (In a current diagnostic apparatus, about two to three waves of sin). In the case shown in Fig. 6A, the space resolution is better, but the discrimination factor between the signal from the contrast agent and a signal from the tissues is worse.

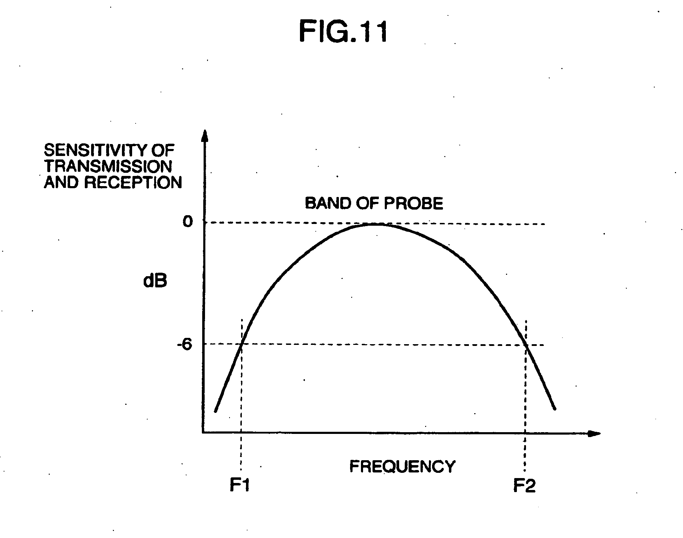

On the one hand, in the case shown in Fig. 6B, the discrimination factor between the signal from the contrast agent and a signal from the tissues is better, but the space resolution is worse. The relative bandwidth of the ultrasonic probe of -6 dB is finite, usually from 60 to 80%. (In addition, in the frequency domain, a lower limit frequency in the range having -6 dB sensitivity from the maximum sensitivity is f1, and an upper limit frequency is f2 (Fig. 11), then -6 dB relative bandwidth is (f2 - f1)/((f1 + f2)/2).) The echo signal from the microbubble already formed of the contrast agent is difficult to be expressed in a simple expression. However, if an exciting signal (that is, a transmission pulse) is long in the time axis, a signal from the contrast agent becomes also long. As shown in Fig. 6B, to discriminate the transmission pulse and the received pulse in the band, if a transmission pulse waveform is made long in the time axis, a received pulse waveform is also long in the time axis, and the space resolution deteriorates.

On the one hand, as shown in Fig. 6A, the received signal is made short in the time axis on a priority basis, and the transmission pulse is also made short in the time axis, then, because the bandwidth of the probe is finite, frequency bands of transmission and reception overlap with each other, resulting in discrimination to be difficult. When in such a manner, after bubble generation, a contrast image is taken, there is a trade off between the discrimination factor of the signal from the contrast agent to a signal from the tissues, and the space resolution, and it is difficult to satisfy both together. In addition, the discrimination factor of a signal A to a signal B may be defined by (an integral of an overlapping portion of bands of two signals)/(an integral of the band of the signal A). Therefore, to improve the discrimination factor, an overlapping portion of signals in the frequency band has to be made small. The emission pulse emitted at the instant of bubble generation, regardless of a pulse length of the transmission waveform, is shortened in the time axis, whereby the discrimination factor and the space resolution both can be enhanced. The emission pulse is shortened in the time axis, because a time length for vaporization is sufficiently short, compared with a cycle of ultrasonic waves (an inverse number of a frequency).

Until now, concerning a pulse length, a long pulse and a short pulse have been described. Now, the long pulse and the short pulse will be hereinafter described quantitatively using specific data.

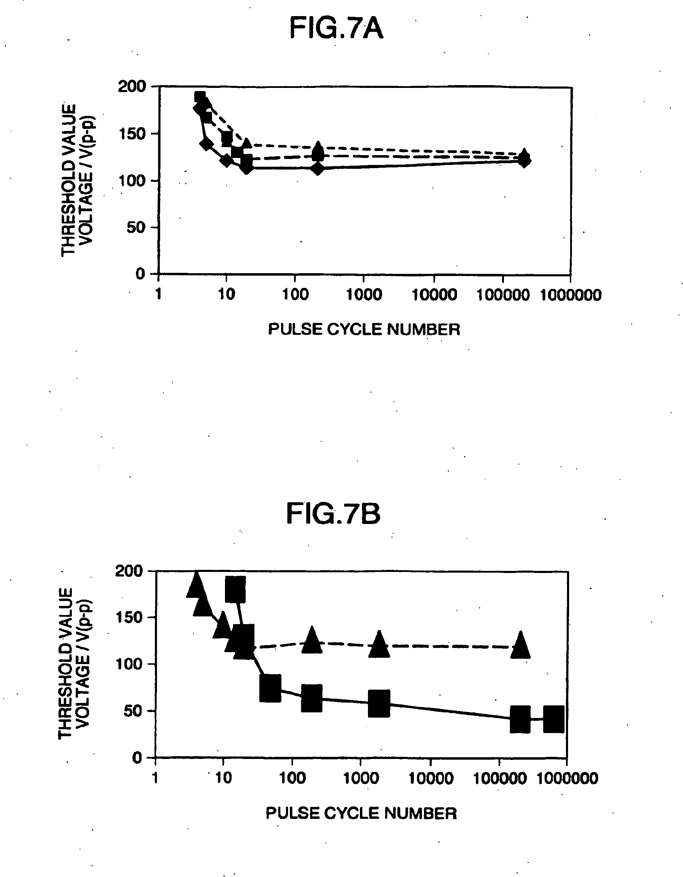

First, from the viewpoint of energy necessary for bubble generation, description will be provided using experimental data. Figs. 7A, 7B show the results where a contrast agent that was a droplet having a diameter of about several hundred nm emulsified from perfluoropentane fixed in acrylamide gel by phospholipids (phosphatidylcholine) and cholesterol was irradiated with ultrasonic waves to obtain a B-mode image, and using the image, a transmission sound pressure caused by bubble generation was evaluated. Fig. 7A shows the relation between a pulse cycle number and a threshold value, when ultrasonic waves of 2.226 MHz were irradiated to determine a threshold voltage for bubble generation using an image of an optical microscope. Fig. 7B shows the relation between the pulse cycle number and the threshold value, when ultrasonic waves of 6.7 MHz or 2.226 MHz were irradiated to determine the threshold voltage for bubble generation using an image of an optical microscope.

A lozenged dot indicates the results in the case where the distribution ratio between perfluoropentane and perfluoroheptane was 100 to 0, which were components of the contrast agent. A rectangular dot indicates the results in the case where the distribution ratio between perfluoropentane and perfluoroheptane was 75 to 25, which were components of the contrast agent. A triangular dot indicates the results in the case where the distribution ratio between perfluoropentane and perfluoroheptane was 50 to 50, which were components of the contrast agent, respectively. In Fig. 7B, a rectangular dot shows the results when a frequency of ultrasonic waves was 6.7 MHz, and the distribution ratio between perfluoropentane and perfluoroheptane was 75 to 25, which were components of the contrast agent. A triangular dot shows the results when a frequency of ultrasonic waves was 2.226 MHz, and also the distribution ratio between perfluoropentane and perfluoroheptane was 75 to 25, which were components of the contrast agent. As shown in Figs. 7A, 7B, the threshold value for bubble generation goes down from some inflection point, as the pulse length is made longer. For example, in the case of the contrast agent used in this experiment, regardless of frequency, beyond 5 $\mu$s, (about 10 pulses at 2.226 MHz and about 100 pulses at 6.7 MHz), there is experimentally shown a threshold value from which the threshold value for bubble generation abruptly lowers. Studying in advance necessary energy for bubble generation for each of such contrast agent and determining a transmission sound pressure, a necessary pulse length is determined, and then the pulse length may be used for the long pulse that has been described.

Next, from the viewpoint of bandwidth, the long pulse will be defined. Fig. 8 is a graph illustrating the relation between the cycle number and the relative bandwidth (bandwidth relative to the center frequency), when the Hanning function is used for an envelope. Here, a pulse waveform of which envelope was the Hanning function was Fourier transformed to compute the relative bandwidth in the frequency domain. The Hanning function, here, will be described as an example, but the envelope may be any function, and by conducting Fourier transformation corresponding to the envelope, a graph similar to Fig. 8 can be obtained. In such a way, studying once the relation between the pulse cycle number and the bandwidth, a window for receiving the long pulse and the emission pulse can be set in the frequency band. The term "window for receiving" used here is an area placed between the phase transition pulse (center frequency fa) and the harmonic component of the phase transition pulse (center frequency fb) in the band of the probe shown in Fig. 4.

For example, when a noise component in the window for receiving of each of the phase transition pulse and the harmonic component thereof is set to be not greater than -6 dB relative to amplitude of a signal at each center frequency, where each -6 dB bandwidth is expressed by $\Delta$fa and $\Delta$fb, respectively, then the window for receiving is in the range from (fa + $\Delta$fa) to (fb - $\Delta$fb). More specifically, for example, the case will be studied where -6 dB band of the probe is from 2 MHz to 4 MHz, and the relative bandwidth is 66%. For example, if the long pulse has 16 waves, and then the relative bandwidth is 12% as shown in Fig. 8. If the long pulse is set to the lower limit of the band of the probe, in the case where the central frequency is set to 2.12 MHz, the long pulse is from 2 to 2.25 MHz, and the harmonic component is from 4 MHz to 4.5 MHz in the case where the bandwidths are approximately the same. Therefore, the band usable for receiving the emission pulse is from 2.25 MHz to 4 MHz and the relative bandwidth is 56% (three to four waves), so that a comparatively better space resolution can be secured. Especially, comparing with the transmission pulse having a considerably long length of 16 waves, it can be seen apparently that the received pulse becomes considerably short having about four waves (from the graph in Fig. 8) and the discrimination factor is better. The discussion, here, has been provided using -6 dB width, but depending on the discrimination factor of a desired signal from a contrast agent to a signal from the tissues, a band may be also studied up to lower sensitivity (for example, -20 dB or -30 dB). Further, the amount of noises from the phase transition pulse and the amount of noises from the harmonic component thereof may be also set separately.

On the contrary to the discussion until now, when the window for receiving the emission pulse is optimized, because, comparing with the space resolution in a current ultrasonic diagnostic apparatus, the space resolution cannot deteriorate much more, the cycle number of the emission pulse is limited to about three waves. In this case, because the relative bandwidth is about 60%, the relative bandwidth obtained by subtracting 60% from the relative bandwidth of the probe can be used for the long pulse. For example, if the relative bandwidth of the probe is 66%, it can be designed so that 6% of the last figure of the relative bandwidth is used for the long pulse, the remaining, 60%, is used for the window for receiving, and the harmonic component of the long pulse is set to be outside the band. In such a way, based on the relative bandwidth necessary for receiving, the transmission pulse for bubble generation can be also designed. (In addition, the reason why an addition and subtraction of the relative bandwidth can be conducted is that, when the whole band f1 to f3 is separated into the band of f1 to f2 and the band of f2 to f3, then each of the separated bandwidths is $(f2 - f1)/(f2 + f1) \times 2$, $(f3 - f2)/(f3 + f2) \times 2$, respectively, and the sum is $(f2 - f1)/(f2 + f1) \times 2 + (f3 - f2)/(f3 + f2) \times 2 = (f3 - f1)/(f3 + f1) \times 2$.

When the emission pulse that is created as the result of transmission of such long pulse is used, there is the degree of freedom where the received pulse is dynamically focused. In the case where a pulse length is quite long, if a timing of emission and a timing of dynamic focusing are out of sync with each other, defocusing may always occur. In the case where a pulse length is long, it may be thought that the dynamic focusing is matched with a time corresponding to the center of the pulse length. In the case where energy immediately before reaching the threshold value for phase transition of the contrast agent is applied, the timing of the dynamic focusing for receiving is matched with approximately the last pulse of the transmission pulse, whereby the transmission of the emission pulse is brought into focus. Here, after the transmission pulse accumulates a constant level of energy, phase transition of the contrast agent is expected to occur, and if the length of the transmission pulse is a little beyond the necessary energy level for this bubble generation, the bubble generation will occur at the last several waves of the transmission pulse. If the dynamic focusing for receiving is matched there, the emission pulse is brought into focus. However, because ultrasonic waves propagate to decay, gradually reducing the sound pressure, the timing of occurrence of bubble generation tends to shift backward in the long pulse, from a shallow place to a deep place. The focal point of the dynamic focusing, considering this point, may be also shifted to some extent in the direction of depth. For example, if the transmission pulse length is ten waves, there is, for example, a method setting so that the sixth wave arrives at a surface of the probe, the tenth wave arrives at a limit in depth, and middle waves between the sixth wave and the tenth wave arrive in between them according to linear interpolation corresponding to a distance.

In addition, this time, the description has been provided using the example of the contrast agent in which the molecular probe and the contrast agent were combined with each other. However, for the contrast agent, a compound that is a fine particle formed of liquid by using a surface active agent can be also used. In this case, for example, if a fine particle having a diameter of 100 to several hundred nm and including an exophthalmos producing reaction (EPR) effect having tumor selectivity dependent on a size thereof is used, the fine particle can be used for a contrast agent for molecular imaging, without the molecular probe combined. The contrast agent used for the present invention may be any substance including material producing a microbubble. Here, the molecular probe may be combined or not.

In this example, the method for detecting only the signal from the contrast agent has been described. However, to display on which portion of a structure of living organisms imaging concentrates, it may be preferable to display a contrast image superimposed on a usual B-mode image. In this case, it is also useful that transmission and reception of the long pulse and ultrasonic waves for imaging the usual B-mode image are alternately repeated to display the B-mode image and the contrast image superimposed with each other.

In addition, by improving selectivity to the emission pulse, it is easy to estimate the amount of agent that generated a microbubble. Further, as the drug delivery system, in the case where an inactivated agent is dosed to the body and the agent is activated for medical care at some timing, the amount of activated agent can be also estimated.

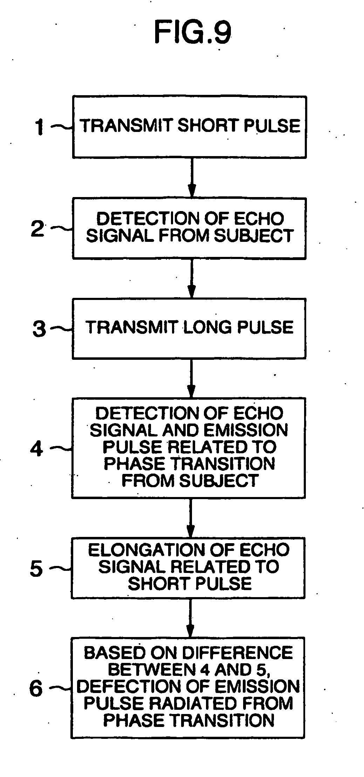

In the following embodiment of the invention, a method for discriminating an echo from the tissues and an emission pulse emitted at the time of bubble generation from the contrast agent, rather than discrimination in the frequency band, will be described. In this embodiment, a short pulse short enough not to generate a microbubble from the contrast agent, and a long pulse to generate a microbubble are used. Because the latter includes the echo from the tissues and the emission pulse, and the former has only the echo from the tissues, by taking a difference between the short pulse and the long pulse, only the emission pulse can be detected. However, because the short pulse and the long pulse are different in waveform, the difference cannot be directly taken. Then, in this embodiment, the short pulse is processed to lengthen pulse after receiving. Long after the completion of transmission and reception of the short pulse, by subtracting from the long pulse including the emission pulse, only the emission pulse can be detected. Fig. 9 shows a flow chart of sequences. First, the short pulse having the cycle number of about one or two similar to that used for taking a usual B-mode image is transmitted and received. After data of one raster is transmitted and received, next, ultrasonic waves of the long pulse as described in the example above is transmitted and received. The echo from the tissues and the emission pulse are discriminated from a received echo upon transmitting the long pulse, as following. It is usually difficult to shorten a pulse in the time axis by using convolution calculation, but it is easy to lengthen it. The convolution calculation is as follows. Where convolution of vectors u and v is w, then it may be expressed as follows.

**[Expression 1]**

$$w(k) = \sum_j u(j)v(k+1-j)$$

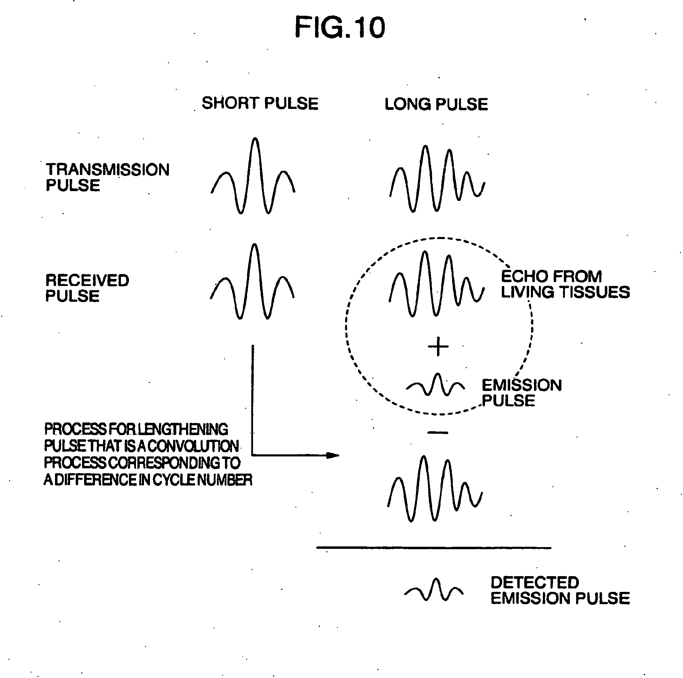

In the present embodiment, for transmission of the short pulse in the first item of the flow chart in Fig. 9, an echo signal received as shown in the second item is processed to have a long wavelength using the convolution calculation. For example, the case where the short pulse has two waves of sin, and the long pulse has twelve waves of sin will be described. In this case, by taking convolution between a waveform having ten waves of sin, that is, an approximate cycle number of a difference between the two cycle numbers, and the original, short pulse, a waveform of about twelve waves of sin is formed. (The convolution calculation, as the expression described above, is a procedure to calculate an integral of an overlapping portion while shifting the two waveforms. Then, shifting the waveforms having widths Ta and Tb back and forth, an interval on which the waveforms overlap is Ta + Tb. In the case where waveforms having two waves of sin and ten waves of sin are convoluted, a waveform having twelve waves of sin is formed). Fig. 10 shows an example of such procedures for lengthening a wavelength. First, a first received pulse of a received signal to a transmission pulse of the short pulse is uniformly processed to lengthen a wavelength.

Next, a second received pulse of a received signal to a transmission pulse of the long pulse is acquired. In the process for lengthening a wavelength described above, it is assumed that the first received pulse is processed to have the same cycle number as that of the second received pulse.

The first received pulse is a signal corresponding to the echo from the tissues. On the one hand, the second received pulse is a signal corresponding to superposition of the echo from the tissues and the emission pulse. Then, by subtracting the first received pulse from the second received pulse, only the emission pulse can be detected.

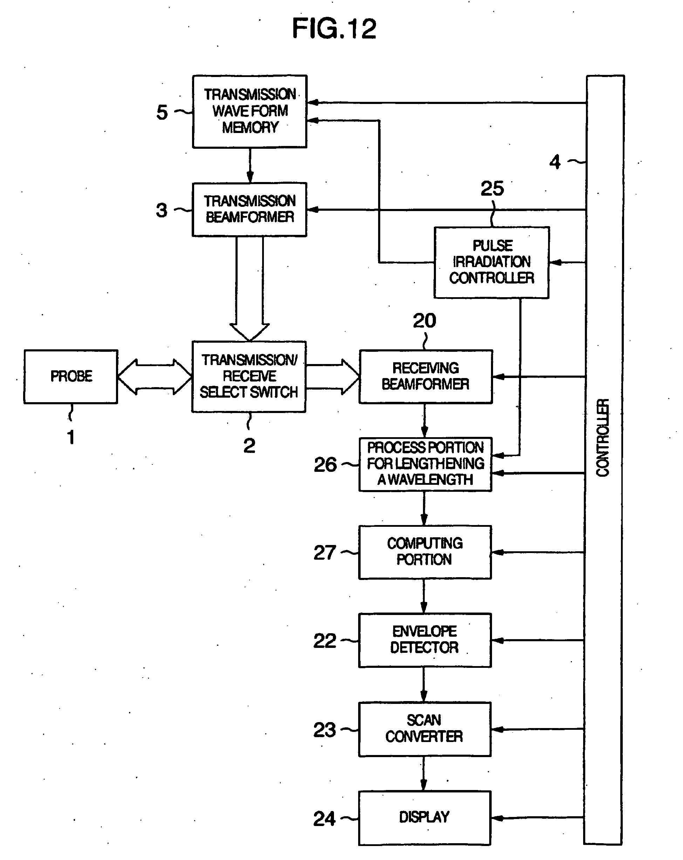

Description using a block diagram of an apparatus is as shown in Fig. 12. Concerning a common portion to Fig. 1, description will be omitted. First, a pulse irradiation controller 25 switches between a long pulse and a short pulse in a transmission waveform memory and a process portion for lengthening a wavelength. An output of a receiving beamformer is processed to lengthen a wavelength as described above by the process portion for lengthening a wavelength 26. By taking a difference between the received signal of the short pulse that underwent this process for lengthening a wavelength and the received signal of the long pulse in a computing portion 27, the emission pulse is detected.

In addition, the process for lengthening a wavelength, more strictly, may be a function that deconvolutes the long pulse by the short pulse, or filter designed using the method of least squares shown as follows. In addition, where a vector C is the result obtained by deconvoluting a vector A by a vector B, then, deconvolution is to find out the vector C so that the vectors B and C are convoluted to form the vector A.

Now, a method for designing a filter to lengthen pulse using a mismatch filter will be hereinafter described. Where a short pulse waveform is B, a filter to lengthen pulse is f, and a signal after lengthening pulse is c, then, the signal after lengthening pulse c may be given by an expression (Expression 2). In the following description, symbols B, W represent matrixes, and c, f, d, w represent vectors, and a mark "T" represents transposition.

**[Expression 2]**

$$c = (f_1, f_2, \ldots f_m) \begin{pmatrix} b_1 & b_2 & \cdots & bn & 0 & \cdots & 0 \\ 0 & b_1 & \ddots & \ddots & \ddots & \ddots & \vdots \\ \vdots & \ddots & \ddots & \ddots & \ddots & \ddots & 0 \\ 0 & \cdots & 0 & b_1 & b_2 & \cdots & bn \end{pmatrix} = fB$$

Let a desired waveform for a signal C to lengthen pulse be D, then the sum I of square error of C and D is (Expression 3). The mismatch filter is a filter F to minimize the sum I of square error.

**[Expression 3]**

$$I = \Sigma \, (c_i - d_i)^2 = (fB - d)(fB - d)^T$$
$$= fBB^Tf^T - dB^Tf^T - fBd^T + dd^T$$

The result obtained by computing (Expression 5) for all i (i = 1, 2, ..., m) under the conditions of (Expression 4) is (Expression 6). The resultant f is (Expression 7).

**[Expression 4]**

$$\partial I/\partial f_i = 0$$

**[Expression 5]**

$$\partial I/\partial f_i = B_iB^Tf^T + fBB_i^T - dB_i^T - B_id^T$$
$$= 2\,(fBB_i^T - dB_i^T) = 0$$

**[Expression 6]**

$$\begin{pmatrix} \dfrac{\partial I}{\partial f_1} \\ \dfrac{\partial I}{\partial f_2} \\ \vdots \\ \dfrac{\partial I}{\partial f_m} \end{pmatrix} = fBB^T - dB^T = 0$$

**[Expression 7]**

$$f = dB^T \, (BB^T)^{-1}$$

Using the filter to lengthen pulse based on a concept of the mismatch filter obtained in such a way, a component of the emission pulse can be more strictly detected.

Here, it is easier to selectively image the emission pulse, when microbubbles are generated from a state in which microbubbles are not present if possible. For that purpose, it may be preferable that a raster of transmission and reception adjacent to each other in terms of time is set to get away from each other as far as possible, compared with the case of scanning the raster in the order from end to end in one direction. For example, when the number of rasters is 100 and they are numbered 1, 2, 3 ..., 100 in the order from the left, an order such as 1, 51, 25, 76, 13, 38, 63, 89, 2, ... may be thought. Doing so, a time interval between adjacent rasters can be lengthened eight times longer than the case of scanning rasters in turn.

It should be further understood by those skilled in the art that although the foregoing description has been made on embodiments of the invention, the invention is not limited thereto and various changes and modifications may be made without departing from the scope of the appended claims.

**Claims**

1. An ultrasonic imaging apparatus, comprising:

   transmission means (1, 2, 3, 5) for transmitting a first ultrasonic signal and a second ultrasonic signal having a cycle number larger than that of the first ultrasonic signal to a phase transition contrast agent dosing area of a subject; and
   receiving means (1, 2, 20) for receiving, from the subject, a third ultrasonic signal and a fourth ultrasonic signal respectively generated by the first ultrasonic signal and the second ultrasonic signal;
   **characterised by** a computing portion (27) for processing the third ultrasonic signal so that a cycle number thereof coincides with that of the second ultrasonic signal, to form a fifth ultrasonic signal, and computing a difference between the fourth ultrasonic signal and the fifth ultrasonic signal as a detection signal.

2. The ultrasonic imaging apparatus according to claim 1, wherein
   the computing portion (27) has a wavelength process portion (26), and
   the wavelength process portion (26) convolutes a pulse having the cycle number of the third ultrasonic signal, and a pulse having a cycle number of a difference between the cycle number of the third ultrasonic signal and the cycle number of the second ultrasonic signal.

3. The ultrasonic imaging apparatus according to claim 1, wherein
   the computing portion (27) has a wavelength process portion (26), and
   the wavelength process portion (26) processes using a function obtained by deconvoluting the second ultrasonic signal by the third ultrasonic signal.

4. The ultrasonic imaging apparatus according to claim 1, wherein
   the computing portion (27) has a wavelength process portion (26), and
   the wavelength process portion (26) processes using a function minimizing square error of the result obtained by convoluting the second ultrasonic signal and the third ultrasonic signal, and the second ultrasonic signal.

5. The ultrasonic imaging apparatus according to claim 1, wherein
   the computing portion (27) has a wavelength process portion (26), and
   the wavelength process portion (26) has a mismatch filter.

6. The ultrasonic imaging apparatus according to claim 1, wherein
   the third ultrasonic signal is a signal corresponding to an echo from tissues, and
   the fourth ultrasonic signal is a signal corresponding to superposition of the echo from the tissues and an emission pulse.

**Patentansprüche**

1. Ultraschall-Bildgebungsvorrichtung aufweisend:

   eine Sende-Einrichtung (1, 2, 3, 5) zum Aussenden eines ersten Ultraschallsignals und eines zweiten Ultraschallsignals mit einer größeren Zykluszahl als das erste Ultraschallsignal an den Dosierbereich für ein Phasenübergangs-Kontrastmittel eines Objekts und
   eine Empfangseinrichtung (1, 2, 20), um vom Objekt ein drittes Ultraschallsignal und ein viertes Ultraschallsignal zu empfangen, die entsprechenderweise vom ersten Ultraschallsignal und vom zweiten Ultraschallsignal verursacht werden,
   **gekennzeichnet durch** einen Berechnungsabschnitt (27), um das dritte Ultraschallsignal so zu verarbeiten, dass seine Zykluszahl mit der des zweiten Ultraschallsignals zusammenfällt, um ein fünftes Ultraschallsignal zu bilden, und um die Differenz zwischen dem vierten Ultraschallsignal und dem fünften Ultraschallsignal als Erfassungssignal zu berechnen.

**2.** Vorrichtung nach Anspruch 1, wobei
der Berechnungsabschnitt (27) einen Wellenlängen-Verarbeitungsabschnitt (26) aufweist und
der Wellenlängen-Verarbeitungsabschnitt (26) einen Impuls mit der Zykluszahl des dritten Ultraschallsignals mit einem Impuls mit der Zykluszahl der Differenz zwischen derjenigen des dritten Ultraschallsignals und derjenigen des zweiten Ultraschallsignals faltet.

**3.** Vorrichtung nach Anspruch 1, wobei
der Berechnungsabschnitt (27) einen Wellenlängen-Verarbeitungsabschnitt (26) aufweist und
der Wellenlängen-Verarbeitungsabschnitt (26) eine Verarbeitung unter Verwendung einer Funktion ausführt, die durch Entfaltung des zweiten Ultraschallsignals durch das dritte Ultraschallsignal gewonnen wird.

**4.** Vorrichtung nach Anspruch 1, wobei
der Berechnungsabschnitt (27) einen Wellenlängen-Verarbeitungsabschnitt (26) aufweist und
der Wellenlängen-Verarbeitungsabschnitt (26) eine Verarbeitung unter Verwendung einer Funktion ausführt, die den quadratischen Fehler zwischen dem zweiten Ultraschallsignal und dem Ergebnis minimiert, das durch Falten des zweiten Ultraschallsignals mit dem dritten Ultraschallsignal gewonnen wird.

**5.** Vorrichtung nach Anspruch 1, wobei
der Berechnungsabschnitt (27) einen Wellenlängen-Verarbeitungsabschnitt (26) aufweist und
der Wellenlängen-Verarbeitungsabschnitt (26) ein Mismatch-Filter aufweist.

**6.** Vorrichtung nach Anspruch 1, wobei
das dritte Ultraschallsignal ein einem Echo von Gewebe entsprechendes Signal ist und
das vierte Ultraschallsignal ein einer Überlagerung des Echos vom Gewebe und eines Emissionsimpulses entsprechendes Signal ist.


**Revendications**

**1.** Dispositif de formation d'images à ultrasons, comportant :

des moyens de transmission (1, 2, 3, 5) pour transmettre un premier signal à ultrasons et un deuxième signal à ultrasons ayant un nombre de cycles plus grand que celui du premier signal à ultrasons à une zone de dosage d'agent de contraste de transition de phase d'un sujet, et
des moyens de réception (1, 2, 20) pour recevoir, en provenance du sujet, un troisième signal à ultrasons et un quatrième signal à ultrasons générés respectivement par le premier signal à ultrasons et le deuxième signal à ultrasons,
**caractérisé par** une partie de calcul (27) pour traiter le troisième signal à ultrasons de sorte qu'un nombre de cycles correspondant coïncide avec celui du deuxième signal à ultrasons, pour former un cinquième signal à ultrasons, et calculer une différence entre le quatrième signal à ultrasons et le cinquième signal à ultrasons en tant que signal de détection.

**2.** Dispositif de formation d'images à ultrasons selon la revendication 1, dans lequel
la partie de calcul (27) a une partie de processus de longueur d'onde (26), et
la partie de processus de longueur d'onde (26) effectue une convolution d'une impulsion ayant le nombre de cycles du troisième signal à ultrasons, et une impulsion ayant un nombre de cycles d'une différence entre le nombre de cycles du troisième signal à ultrasons et le nombre de cycles du deuxième signal à ultrasons.

**3.** Dispositif de formation d'images à ultrasons selon la revendication 1, dans lequel
la partie de calcul (27) a une partie de processus de longueur d'onde (26), et
la partie de processus de longueur d'onde (26) traite en utilisant une fonction obtenue par déconvolution le deuxième signal à ultrasons par l'intermédiaire du troisième signal à ultrasons.

**4.** Dispositif de formation d'images à ultrasons selon la revendication 1, dans lequel
la partie de calcul (27) a une partie de processus de longueur d'onde (26), et
la partie de processus de longueur d'onde (26) traite en utilisant une fonction minimisant une erreur carrée du résultat obtenu par convolution le deuxième signal à ultrasons et le troisième signal à ultrasons, et le deuxième signal à ultrasons.

**5.** Dispositif de formation d'images à ultrasons selon la revendication 1, dans lequel
la partie de calcul (27) a une partie de processus de longueur d'onde (26), et
la partie de processus de longueur d'onde (26) a un filtre de défaut d'adaptation d'impédance.

**6.** Dispositif de formation d'images à ultrasons selon la revendication 1, dans lequel
le troisième signal à ultrasons est un signal correspondant à un écho provenant de tissus, et
le quatrième signal à ultrasons est un signal correspondant à une superposition de l'écho provenant des tissus et
d'une impulsion d'émission.

# FIG.1

## FIG.2A

```
┌─────────────────────┐
│    DOSING OF        │
│  CONTRAST AGENT     │
└─────────────────────┘
           │
           ▼
┌─────────────────────┐
│  PHASE TRANSITION   │
│ OF CONTRAST AGENT   │
└─────────────────────┘
           │
           ▼
┌─────────────────────┐
│    IMAGING OF       │
│   MICRO BUBBLE      │
│   (PULSE ECHO)      │
└─────────────────────┘
```

## FIG.2B

```
┌─────────────────────────────────────┐
│              PROBE                   │
└─────────────────────────────────────┘
```

FUNDAMENTAL
WAVE

FUNDAMENTAL
ECHO

ECHO FROM
BUBBLE

HARMONIC
ECHO

BUBBLE

# FIG.3A

DOSING OF
CONTRAST AGENT

PHASE TRANSITION
OF CONTRAST AGENT
& IMAGING BASED ON
EMISSION SIGNAL
WHEN BUBBLE IS
GENERATED

# FIG.3B

PROBE

PHASE
TRANSITION PULSE

ECHO RELATED
TO PHASE
TRANSITION PULSE

EMISSION
PULSE

HARMONIC ECHO
RELATED TO PHASE
TRANSITION PULSE

BUBBLE
GENERATION

# FIG.4

TIME DOMAIN

NARROW BAND PHASE
TRANSITION PULSE

EMISSION PULSE

BAND OF PROBE

FREQUENCY DOMAIN

HARMONIC
COMPONENT
OF PHASE
TRANSITION
PULSE

FREQUENCY

fa

fb

# FIG.5

```
┌─────────────────────────┐
│     TRANSMISSION OF      │
│     LONG PULSE FOR       │
│    PHASE TRANSITION      │
└─────────────────────────┘
             │
             ▼
┌─────────────────────────┐
│    DETECTION OF ECHO     │
│  SIGNAL AND EMISSION     │
│    PULSE RELATED TO      │
│   PHASE TRANSITION       │
│     FROM SUBJECT         │
└─────────────────────────┘
             │
             ▼
┌─────────────────────────┐
│ DETECTION OF EMISSION    │
│   PULSE RELATED TO       │
│   PHASE TRANSITION       │
│ USING BAND PASS FILTER   │
└─────────────────────────┘
```

EP 1 965 224 B1

# FIG.6A     FIG.6B

**FIG.6A**

IT IS DIFFICULT TO
DISCRIMINATE
BETWEEN TISSUE
ECHO AND
CONTRAST ECHO

TRANSMISSION   RECEPTION

FREQUENCY
CHARACTERISTIC

TRANSMISSION
PULSE

RECEIVED
PULSE

HIGH SPACE
RESOLUTION

**FIG.6B**

IT IS EASY TO
DISCRIMINATE
BETWEEN TISSUE
ECHO AND
CONTRAST ECHO

TRANSMISSION   RECEPTION

LOW SPACE
RESOLUTION

## FIG.7A

## FIG.7B

# FIG.8

# FIG.9

1 — TRANSMIT SHORT PULSE

2 — DETECTION OF ECHO SIGNAL FROM SUBJECT

3 — TRANSMIT LONG PULSE

4 — DETECTION OF ECHO SIGNAL AND EMISSION PULSE RELATED TO PHASE TRANSITION FROM SUBJECT

5 — ELONGATION OF ECHO SIGNAL RELATED TO SHORT PULSE

6 — BASED ON DIFFERENCE BETWEEN 4 AND 5, DEFECTION OF EMISSION PULSE RADIATED FROM PHASE TRANSITION

# FIG.10

SHORT PULSE   LONG PULSE

TRANSMISSION
PULSE

RECEIVED
PULSE

ECHO FROM
LIVING TISSUES

+

EMISSION
PULSE

PROCESS FOR LENGTHENING
PULSE THAT IS A CONVOLUTION
PROCESS CORRESPONDING TO
A DIFFERENCE IN CYCLE NUMBER

−

DETECTED
EMISSION PULSE

# FIG.11

SENSITIVITY OF
TRANSMISSION
AND RECEPTION

BAND OF PROBE

0

dB

-6

FREQUENCY

F1    F2

# FIG.12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6521211 B
- US 5716597 A
- JP 11137547 A
- JP 2003135467 A
- US 5833614 A
- US 20050273010 A
- EP 1723911 A

### Non-patent literature cited in the description

- **ELLEGALA et al.** *Circulation,* 2003, vol. 108, 336-341
- **LANZA et al.** *Circulation,* 1996, vol. 94, 3334-3340
- **Stride et al.** Microbubble ultrasound contrast agents: a review. *Proceedings of the Institution of Mechanical Engineers, Journal of Engineering in Medicine,* November 2003, vol. 217 (6), 429-447